# EUROPÄISCHE PATENTSCHRIFT

(11) **EP 1 232 151 B1**
(45) Veröffentlichungstag und Bekanntmachung des Hinweises auf die Patenterteilung: **09.07.2003**
(21) Anmeldenummer: 00977470.4
(22) Anmeldetag: 03.11.2000
(51) Int. Cl.: C07D 307/82, C07D 307/83

(54) **VERFAHREN ZUR HERSTELLUNG VON BENZOFURANONOXIMEN**
METHOD FOR PRODUCTION OF BENZOFURANONE OXIMES
PROCEDE DE PREPARATION DE BENZOFURANONOXIMES

(30) Priorität: 16.11.1999 DE 19954936
(43) Veröffentlichungstag der Anmeldung: 21.08.2002
(73) Patentinhaber: Bayer CropScience AG, 40789 Monheim (DE)
(72) Erfinder: LANTZSCH, Reinhard, 42115 Wuppertal (DE); GAYER, Herbert, 40789 Monheim (DE); GERDES, Peter, 52080 Aachen (DE); HÜBSCH, Walter, 42113 Wuppertal (DE); MULDER, Lubbertus, 58135 Hagen-Haspe (DE); GALLENKAMP, Bernd, 42113 Wuppertal (DE); DECKER, Matthias, 50674 Köln (DE)
(86) Internationale Anmeldenummer: EP0010826
(87) Internationale Veröffentlichungsnummer: WO01036405

(56) Entgegenhaltungen:
- EP-A- 0 846 691
- F. W. LICHTENTHALER ET AL.: "SELECTIVE DEACETYLATION ..." TETRAHEDRON., Bd. 21, 1980, Seiten 1425-1428, XP002163748 ELSEVIER SCIENCE PUBLISHERS, AMSTERDAM., NL ISSN: 0040-4020
- A. R. KATRITZKY, A. J. BOULTON: "Advances in Heterocyclic Chemistry, Vol. 18" 1975 , ACADEMIC PRESS , NEW YORK XP002163750 in der Anmeldung erwähnt Verbindungen 282 und 283 Seite 434 -Seite 435
- C. F. CARVALHO ET AL.: "Naturally occuring dibenzofurans. ..." JOURNAL OF THE CHEMICAL SOCIETY, PERKIN TRANSACTIONS 1., 1984, Seiten 1605-1612, XP002163749 CHEMICAL SOCIETY. LETCHWORTH., GB ISSN: 1472-7781 in der Anmeldung erwähnt

## Beschreibung

Die vorliegende Anmeldung betrifft ein neues Verfahren zur Herstellung von bekannten Benzofuranonoximen, sowie ein neues Verrahren zur Herstellung von bekannten Benzofuranylalkanolaten, die als Zwischenprodukte in der Synthesesequenz Verwendung finden.

Es ist bereits bekannt geworden, dass man Benzofuranonoxime der Formel (D) nach folgender Synthesesequenz erhalten kann:

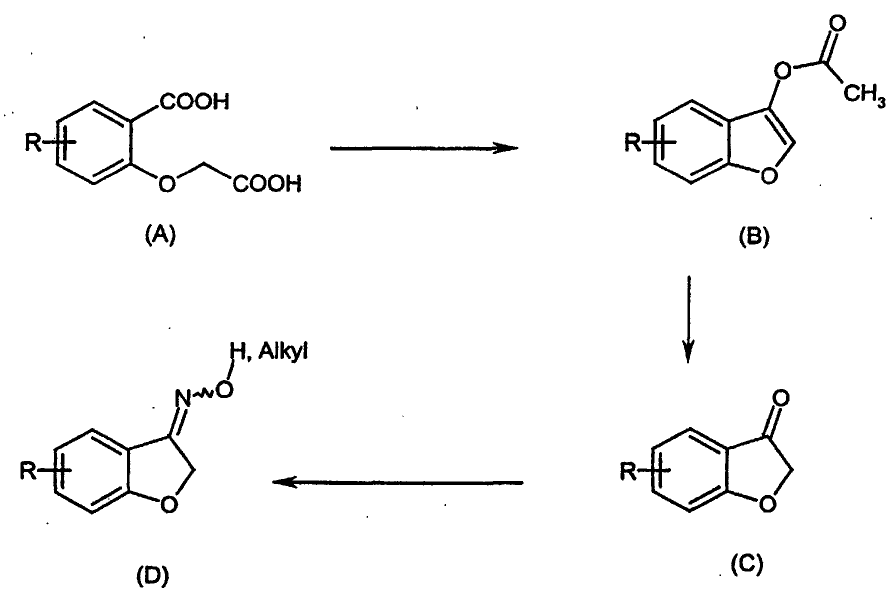

Im ersten Schritt der Synthese wird ein Salicylsäurederivat der Formel (A) (vergleiche z. B. J. Chem. Soc. 1932, 1380; Chem. Ber. 33, 1398, (1900); Chem. Ber. 46, 3370, (1913)) mit Acetanhydrid und Natriumacetat, gegebenenfalls in Gegenwart von Eisessig, erhitzt (vergleiche z. B. Adv. Het. Chem. 18, 434 und J. Chem. Soc. Perkin Trans, I, 1984, 1605-1612). Der Nachteil der beschriebenen Verfahren liegt zum einen in den unbefriedigenden Ausbeuten an entsprechendem Benzofuranylacetat der Formel (B) und insbesondere an dem bei der Reaktion entstehenden Nebenprodukt der Formel (E):

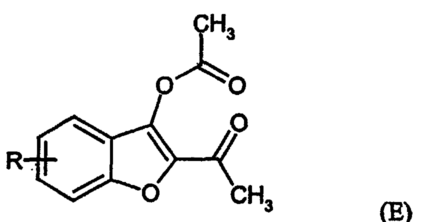

Dieses Nebenprodukt entsteht in Anteilen bis zu 10 %, ist vom gewünschten Produkt nur sehr schwer abtrennbar und ist damit ein ganz besonderer Nachteil der bekannten Verfahren zur Herstellung von Benzofuranylacetaten der Formel (B).

Im nächsten Schritt werden die Benzofuranylacetate der Formel (B) zu den Benzofiranonen der Formel (C) hydrolysiert (vergleiche z. B. J. Chem. Soc. Perkin Trans, I, 1984, 1609). Auch in diesem Schritt werden nur unbefriedigende Ausbeuten erhalten. Weiterhin sind die Benzofuranone der Formel (C) chemisch instabil und nicht lagerfähig (vergleiche z. B. The Chemistry ofHeterocyclic Compounds, 29, 226).

Die Synthese der Benzofuranonoxime der Formel (D) aus den Benzofuranonen der Formel (C) und entsprechenden Hydroxylaminderivaten ist ebenfalls beschrieben (vergleiche z. B. Chem. Ber. 33, 3178 (1900) und EP-A 846691). Der Nachteil dieses Verfahrensschrittes liegt im Einsatz der instabilen Bemzofuranone als Ausgangsstoffe.

Es wurde nun gefunden, dass man
a) Benzofuranonoxime der Formel (I), 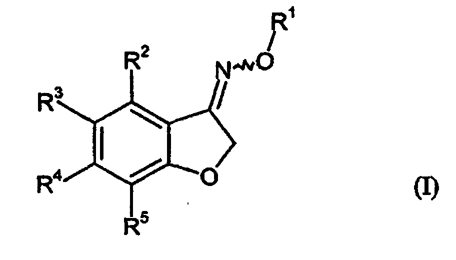 in welcher
   - R¹: für Wasserstoff oder Alkyl steht und
   - R², R³, R⁴ und R⁵: gleich oder verschieden sind und unabhängig voneinander für Wasserstoff, Alkyl, Alkoxy, Halogenalkyl oder Halogen steht,
   erhält, wenn man Benzofuranylalkanolate der allgemeinen Formel (II), 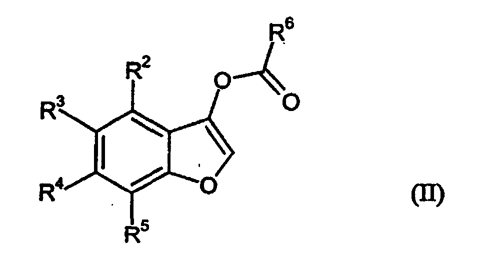 in welcher
   - R², R³, R⁴ und R⁵: die oben angegebenen Bedeutungen haben und
   - R⁶: für Alkyl steht,
   mit einer Säure, gegebenenfalls in Gegenwart eines Verdünnungsmittels, hydrolysiert und das dabei erhaltene Benzofuranon der Formel (III), 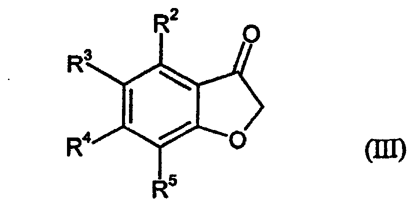 in welcher
   - R², R³, R⁴ und R⁵: die oben angegebenen Bedeutungen haben,
   ohne Aufarbeitung mit einem Hydroxylaminderivat der Formel (IV),

   H₂N-O-R¹ (IV)

   in welcher
   - R¹: die oben angegebene Bedeutung hat,
   oder einem Säureadditionskomplex davon,
   gegebenenfalls in Gegenwart eines Verdünnungsmittels und gegebenenfalls in Gegenwart eines Puffermediums, umsetzt.
   Die bei der Durchführung des erfindungsgemäßen Verfahrens als Ausgangsstoffe benötigten Benzofüranylalkanolaten sind durch die Formel (II) allgemein definiert, bevorzugt einsetzbar sind Verbindungen der Formel (II), in welcher
   - R², R³, R⁴ und R⁵: gleich oder verschieden sind und unabhängig voneinander für Wasserstoff, Alkyl oder Alkoxy mit jeweils 1 bis 4 Kohlenstoffatomen, Halogenmethyl oder Halogen stehen und
   - R⁶: für Alkyl steht.

   Besonders bevorzugt ist die Herstellung von Benzofuranylalkanolaten der Formel (II), in welcher
   - R², R³, R⁴ und R⁵: gleich oder verschieden sind und unabhängig voneinander für Wasserstoff, Methyl, Methoxy, t-Butyl, Trifluormethyl, Fluor, Chlor oder Brom steht, wobei ganz besonders bevorzugt mindestens drei der Substituenten R² bis R⁵ für Wasserstoff stehen und
   - R⁶: für Methyl, Ethyl, n- oder i-Propyl, n-, i-, s- oder t-Butyl steht,

   R⁶ steht im allgemeinen besonders bevorzugt für Methyl oder Ethyl.
   Die Benzofuranylalkanolate der Formel (II) sind bekannt und können nach bekannten Methoden erhalten werden (vergleiche z. B. Adv. Het. Chem. 18, 434 und J. Chem. Soc. Perkin Trans, I, 1984, 1605-1612).
   Es ist als ausgesprochen überraschend zu bezeichnen, dass das als Zwischenprodukt in der Reaktionsmischung entstehende Benzofuranon nicht, wie in der Literatur beschrieben, teilweise einer Zersetzung in Form einer Oxidation oder Polymerisation unterliegt, sondern sofort mit dem Hydroxylaminderivat der Formel (IV) zum gewünschten Endprodukt in sehr hoher Reinheit abreagiert.
   Das erfindungsgemäße Verfahren a) weist eine Reihe von Vorteilen auf. So lassen sich Benzoniranonamine bei hohen Ausbeuten und Reinheiten aus leicht zugänglichen Ausgangsstoffen unter technisch problemlosen Bedingungen erhalten.
   Die weiterhin als Ausgangsstoffe zur Durchführung des erfindungsgemäßen Verfahrens a) benötigten Hydroxylaminderivate, bzw. deren Säureadditionskomplexe sind durch die Formel (IV) allgemein definiert. In dieser Formel (IV) steht R¹ für Wasserstoff oder Methyl. Bevorzugte Säureadditionskomplexe von Verbindungen der Formel (IV) sind die Hydrochloride bzw. die Hydrogensulfate oder Sulfate.
   Als Verbindung der Formel (IV) wird O-Methylhydroxylamin bevorzugt. Besonders bevorzugt ist Hydroxylamin oder dessen Säureadditionskomplexe.
   Die Hydroxylaminderivate der Formel (IV), bzw. deren Säureadditionskomplexe sind bekannte Synthesechemikalien.
   Es wurde ferner gefunden, dass man
b) Benzofuranylalkanolate der allgemeinen Formel (II) erhält, wenn man Salicylsäurederivate der Formel (V), 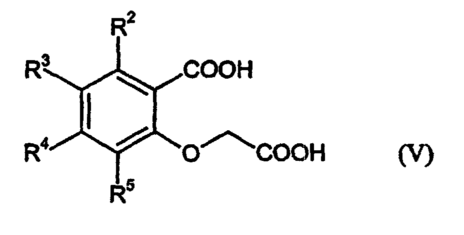 in welcher
   R², R³, R⁴, R⁵ die oben angegebenen Bedeutungen haben
   mit aliphatischen Säureanhydriden der Formel (VI), 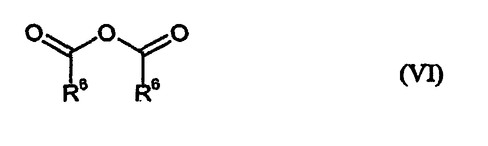 in welcher
   - R⁶: die oben angegebene Bedeutung hat,
   oder aliphatischen Säurechloriden der Formel (VII), 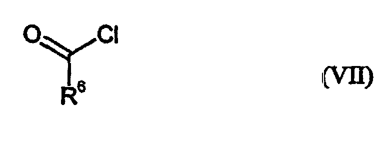 in welcher
   - R⁶: die oben angegebene Bedeutung hat,
   in Gegenwart eines Katalysators umsetzt.

Es ist ausgesprochen überraschend zu bewerten, dass die Bildung des in der Literatur beschriebenen, störenden und schwer entfernbaren Nebenproduktes praktisch völlig unterdrückt wird.

Das erfindungsgemäße Verfahren b) weist eine Reihe von Vorteilen auf. So lassen sich Benzofuranylalkanolate in hohen Ausbeuten und Reinheiten aus leicht zugänglichen Ausgangsstoffen unter technisch völlig problemlosen Bedingungen erhalten.

Die zur Durchführung des erfindungsgemäßen Verfahrens b) als Ausgangsstoffe benötigten Salicylsäurederivate sind durch die Formel (V) allgemein definiert. In dieser Formel (V) haben R², R³, R⁴ und R⁵ vorzugsweise, bzw. insbesondere diejenigen Bedeutungen, die im Zusammenhang mit der Beschreibung der Verbindungen der Formel (II) als bevorzugt, bzw. als insbesondere bevorzugt für R², R³, R⁴ und R⁵ angegeben wurden.

Die Salicylsäurederivate der allgemeinen Formel (V) sind bekannt und können nach bekannten Methoden erhalten werden (vergleiche z. B. J.Chem. Soc. 1932, 1380; Chem. Ber. 33, 1398, (1900); Chem. Ber. 46, 3370, (1913)).

Die weiterhin als Ausgangsstoffe zur Durchführung des erfindungsgemäßen Verfahrens b) benötigten Carbonsäureanhydride sind durch die Formel (VI) allgemein definiert. In dieser Formel (VI) hat R⁶ vorzugsweise, bzw. insbesondere diejenige Bedeutung, die im Zusammenhang mit der Beschreibung der Verbindungen der Formel (IV) als bevorzugt, bzw. als insbesondere bevorzugt flir R⁶ angegeben wurde.

Die weiterhin alternativ als Ausgangsstoffe zur Durchführung des erfindungsgemäßen Verfahrens b) benötigten Carbonsäurechloride sind durch die Formel (VII) allgemein definiert. In dieser Formel (VII) hat R⁶ vorzugsweise, bzw. insbesondere diejenige Bedeutung, die im Zusammenhang mit der Beschreibung der Verbindungen der Formel (II) als bevorzugt, bzw. als insbesondere bevorzugt für R⁶ angegeben wurde.

Die Carbonsäureanhydride der Formel (VI), bzw. die Carbonsäurechloride der Formel (VII) sind bekannte Synthesechemikalien.

Als Verdünnungsmittel zur Durchführung der ersten und der zweiten Stufe des erfindungsgemäßen Verfahrens a) kommen inerte organische Lösungsmittel in Frage. Hierzu gehören beispielhaft und vorzugsweise Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; oder Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Als Verdünnungsmittel zur Durchführung der ersten und der zweiten Stufe des erfindungsgemäßen Verfahrens a) werden vorzugsweise Alkohole, insbesondere Ethanol, besonders bevorzugt Methanol verwendet.

Die erste Stufe des erfindungsgemäßen Verfahrens a) wird in Gegenwart einer Säure durchgeführt. Als solche kommen alle anorganischen und organischen Protonensäuren, sowie auch alle polymeren Säuren infrage. Hierzu gehören beispielhaft und vorzugsweise Chlorwasserstoff, Schwefelsäure, Phosphorsäure, Ameisensäure, Essigsäure, Trifluoressigsäure, Methansulfonsäure, Trifluonnethansulfonsäure, Toluolsulfonsäure, saure Ionenaustauscher, saure Tonerden und saures Kieselgel. Bevorzugte Säuren sind Salzsäure und Schwefelsäure.

Die zweite Stufe des erfindungsgemäßen Verfahrens a) wird gegebenenfalls in Gegenwart eines Puffermediums durchgeführt. Bevorzugt ist ein pH-Wert von 3 bis 7. Als Puffermedien kommen alle üblichen Säure/Salzgemische infrage, die den pH-Wert in diesem Bereich puffern. Bevorzugt ist das Gemisch Essigsäure/Natriumacetat. In einer besonderen Ausführungsform wird, nachdem die erste Stufe des erfindungsgemäßen Verfahrens a) abreagiert hat, so viel Natriumacetat und/oder Natronlauge zu der Reaktionsmischung gegeben, dass die Mischung den gewünschten pH-Wert erreicht hat.

Die Reaktionstemperaturen können bei der Durchführung der ersten Stufe des erfindungsgemäßen Verfahrens a) in einem größeren Bereich variiert werden. Im allgemeinen arbeitet man bei Temperaturen von 20°C bis 120°C, vorzugsweise bei Temperaturen von 40°C bis 100°C.

Die zweite Stufe des erfindungsgemäßen Verfahrens a) wird im allgemeinen bei Temperaturen von 0°C bis 80°C, vorzugsweise bei Temperaturen von 20°C bis 60°C durchgeführt.

Die erste und zweite Stufe des erfindungsgemäßen Verfahrens a) werden im allgemeinen unter Normaldruck durchgeführt. Es ist jedoch auch möglich, unter erhöhtem Druck - im allgemeinen bis zu 10 bar - zu arbeiten.

Zur Durchführung des erfindungsgemäßen Verfahrens a) zur Herstellung der Verbindungen der Formel (I) setzt man pro Mol des Benzofuranylalkanolats der Formel (II) im allgemeinen 1 bis 5 Mol, vorzugsweise 1 bis 2 Mol Hydroxylaminderivat der Formel (IV) ein.

Zur Durchführung des erfindungsgemäßen Verfahrens a) geht man im allgemeinen wie folgt vor. Das Benzofuranylalkanolat der Formel (II) wird vorzugsweise in Gegenwart eines Verdünnungsmittels mit einer Säure versetzt und anschließend erhitzt. Nach beendeter Hydrolyse wird die Mischung abgekühlt, vorzugsweise auf eine Temperatur von 35 bis 45°C, mit dem Hydroxylamnnderivat der Formel (IV), bzw. dessen Säureadditionskomplex, und dem Puffermedium versetzt und wiederum erwärmt. Der pH-Wert wird gegebenenfalls durch Zugabe von einer Base, beispielsweise Natronlage, genau eingestellt. Nach beendeter Reaktion wird in üblicher Weise aufgearbeitet. Man destilliert beispielsweise flüchtige Lösungsmittelanteile ab und versetzt mit Wasser, wobei das Produkt kristallisiert.

Als Verdünnungsmittel zur Durchführung des Verfahrens b) unter Verwendung eines Säureanhydrids der Formel (VI) werden als Verdünnungsmittel inerte organische Lösungsmittel verwendet oder bevorzugt kein Lösungsmittel verwendet.

Als Verdünnungsmittel zur Durchführung des Verfahrens b) unter Verwendung eines Säurechlorids der Formel (VII) werden als Verdünnungsmittel inerte organische Lösungsmittel verwendet. Vorzugsweise werden aromatische Kohlenwasserstoffe, wie Toluol oder Chlorbenzol verwendet.

Das erfindungsgemäße Verfahren b) wird vorzugsweise in Gegenwart eines geeigneten Katalysators durchgeführt. Als solche kommen alle üblichen organischen Basen infrage. Hierzu gehören vorzugsweise tertiäre Amine, wie Trimethylamin, Triethylamin, Tributylamin, N,N-Dimethylanilin, N,N-Dimethyl-benzylamin, Pyridin, Picoline, 2-Mehtyl-5-ethyl-pyridin, N-Methylpiperidin, N-Methylmorpholin, N,N-Dimethylaminopyridin, Diazabicyclo octan (DABCO), Diazabicyclononen (DBN) oder Diazabicycloundecen (DBU). Bevorzugt sind Pyridin, N,N-Dimethylaminopyridin, Picoline und 2-Methyl-5-ethyl-pyridin. Besonders bevorzugt sind Pyridinderivate, insbesondere Pyridin.

Die Reaktionstemperaturen können bei der Durchführung des erfindungsgemäßen Verfahrens b) in einem größeren Bereich variiert werden. Im allgemeinen arbeitet man bei Temperaturen von 80°C bis 200°C, bevorzugt bei Temperaturen von 120°C bis 150°C, besonders bevorzugt vorzugsweise bei Temperaturen von 130°C bis 140°C.

Das erfindungsgemäße Verfahren b) wird im allgemeinen unter Normaldruck durchgeführt. Es ist jedoch auch möglich, unter erhöhtem Druck - im allgemeinen bis zu 10 bar - zu arbeiten.

Zur Durchführung des erfindungsgemäßen Verfahrens b) zur Herstellung der Verbindungen der Formel (II) setzt man pro Mol des Salicylsäurederivates der Formel (V) im allgemeinen 2 bis 10 Mol, vorzugsweise 4 bis 6 Mol Carbonsäureanhydrid der Formel (VI) oder Carbonsäurechlorid der Formel (VII) ein.

Zur Durchführung des erfindungsgemäßen Verfahrens b) geht man im allgemeinen wie folgt vor. Das Salicylsäurederivat der Formel (V) wird mit dem Carbonsäureanhydrid der Formel (VI), bzw. dem Carbonsäurechlorid der Formel (VII) und dem Katalysator gemischt und erhitzt. Setzt man ein Carbonsäureanhydrid der Formel (VI) ein, kann es von Vorteil sein, die während der Reaktion enstehende Carbonsäure über eine Destillationskolonne zu entfernen. Nach beendeter Reaktion wird in üblicher Weise aufgearbeitet. Man destilliert beispielsweise die leichter flüchtigen Bestandteile aus der Reaktionsmischung bei schwach vermindertem Druck ab und destilliert anschließend das Produkt bei stark vermindertem Druck.

In einer besonders bevorzugten Verfahrensvariante wird das Benzofuranylalkanolat der Formel (II) nicht isoliert, sondern nach dem Abdestillieren der flüchtigen Bestandteile aus Verfahren b) gleich mit einem Verdünnungsmittel und einer Säure versetzt und anschließend erhitzt, wie bei Verfahren a) beschrieben. Die weitere Reaktionsdurchführung und Aufarbeitung erfolgt wie bei Verfahren a). Bei dieser Variante ist es von besonderem Vorteil, dass auf die Reinigung des Benzofuranylalkanolates der Formel (II) verzichtet werden kann.

Die Benzofuranonoxime der Formel (I) können als Vorstufen zur Herstellung von Fungiziden verwendet werden (vergleiche z. B. EP-A 846 691).

Die nachfolgenden Beispiele dienen zur Erläuterung der Erfindung. Die Erfindung ist jedoch nicht auf die Beispiele limitiert.

### Herstellungsbeispiele:

### Beispiel 1

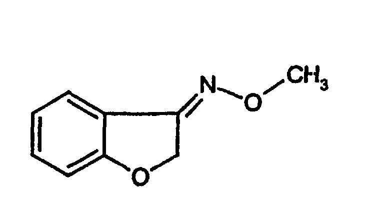

17,6 g (0,1 Mol) 3-Acetoxy-benzofuran werden in 100 ml Dimethoxyethan gelöst und 6 ml 20 % ige Salzsäure zugegeben. Man erhitzt unter Rühren zwei Stunden auf 65° C, lässt auf ca. 30-35° C abkühlen und lässt 33,4 g (0,12 Mol) 30 % ige wässrige Lösung von O-Methylhydroxylaminhydrochlorid zulaufen. Dann gibt man 12 g Natriumacetat zu und erwärmt 4 Stunden auf 40-45°C. Das Lösungsmittel wird unter vermindertem Druck abdestilliert, die Wasserphase noch etwas verdünnt und dreimal mit Methylenchlorid extrahiert. Man erhält 14,2 g Benzofuran-O-methyloxim, das beim Stehen langsam zu kristallisieren beginnt.
Fp: 38 °C, log P 2,38
Ausbeute: 87,0 % d.Th.

### Beispiel 2

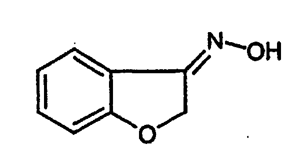

35,2 g (0,2 Mol) 3-Acetoxy-benzofuran werden in 180 ml Methanol gelöst, 9 ml Wasser und 4,9 g 20 % ige Salzsäure zugegeben und 2 Stunden zum Sieden erhitzt. Man kühlt auf 35° C ab, gibt 23,9 g (0,284 Mol) Natriumacetat zu und anschließend 15,4 g (0,22 Mol) Hydroxylammoniumchlorid. Man erhitzt unter Rühren 4,5 Stunden auf 45° C, lässt abkühlen und destilliert das Methanol ab. Der Rückstand wird mit 220 ml Wasser versetzt und der Feststoff abfiltriert. Man wäscht mit Wasser, dann mit Toluol und trocknet im Vakuumtrockenschrank. Man erhält 28,2 g schwach gefärbte Kristalle mit einem Gehalt von 97 %, enspricht einer Ausbeute von 91,7 % d.Th.
Fp: 157-159°C, log P 1,44

### Beispiel 3

### Verfahren (b)

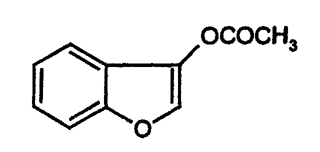

58,9 g (0,3 Mol) 2-Carboxy-phenoxyessigsäure, 153 g (1,5 Mol) Acctanhydrid und 1,2 g (0,015 Mol) Pyridin werden zusammengegeben und zum Sieden erhitzt. Nach 24 Stunden kühlt man ab, destilliert Essigsäure und überschüssiges Acetanhydrid ab und entfernt die letzten flüchtigen Bestandteile bei 35° C / 5mbar.

Man erhält 52,8 g eines Öls, das direkt in die nächste Stufe eingesetzt werden kann. Gehalt: 97 %, Ausbeute: 96,9 % d.Th..

Eine Reinigung durch Destillation ist nicht notwendig, aber möglich: Kp = 85° C / 0,05 mbar.

Analog dem Beispiel 3 lassen sich folgende Verbindungen herstellen:

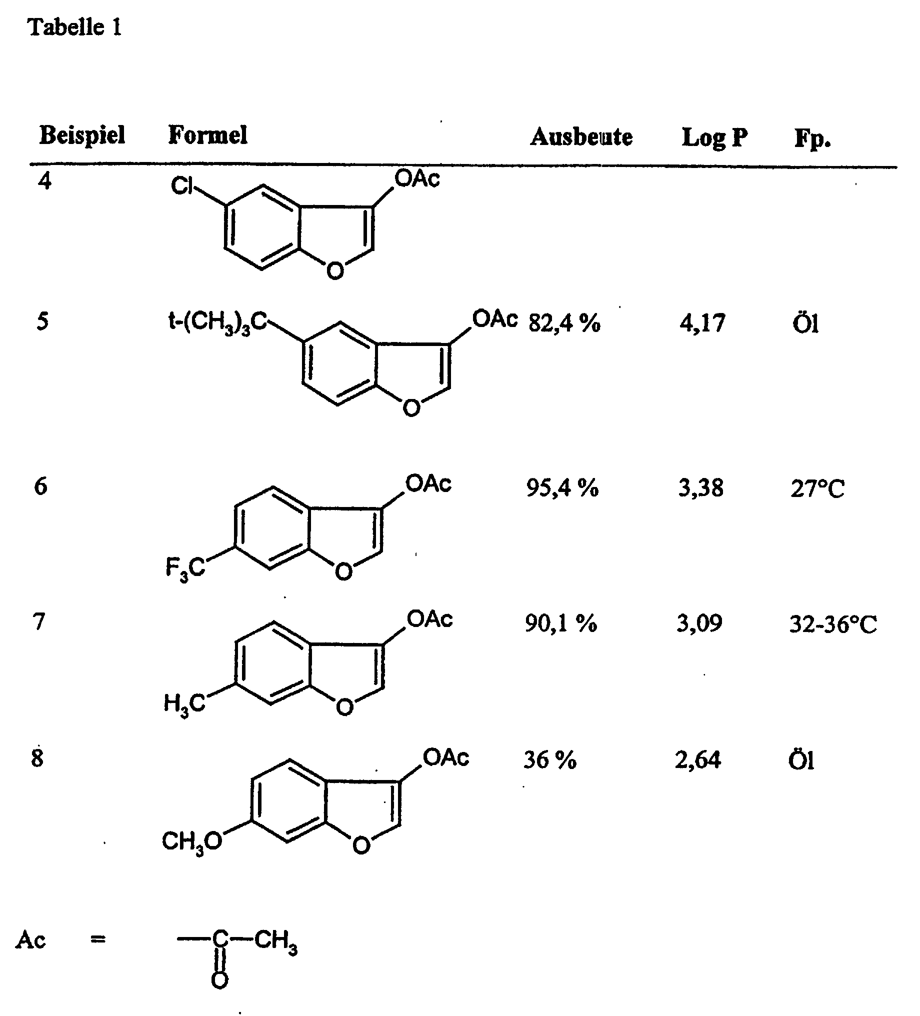

Analog Beispiel 2 lassen sich die folgenden Verbindungen herstellen:

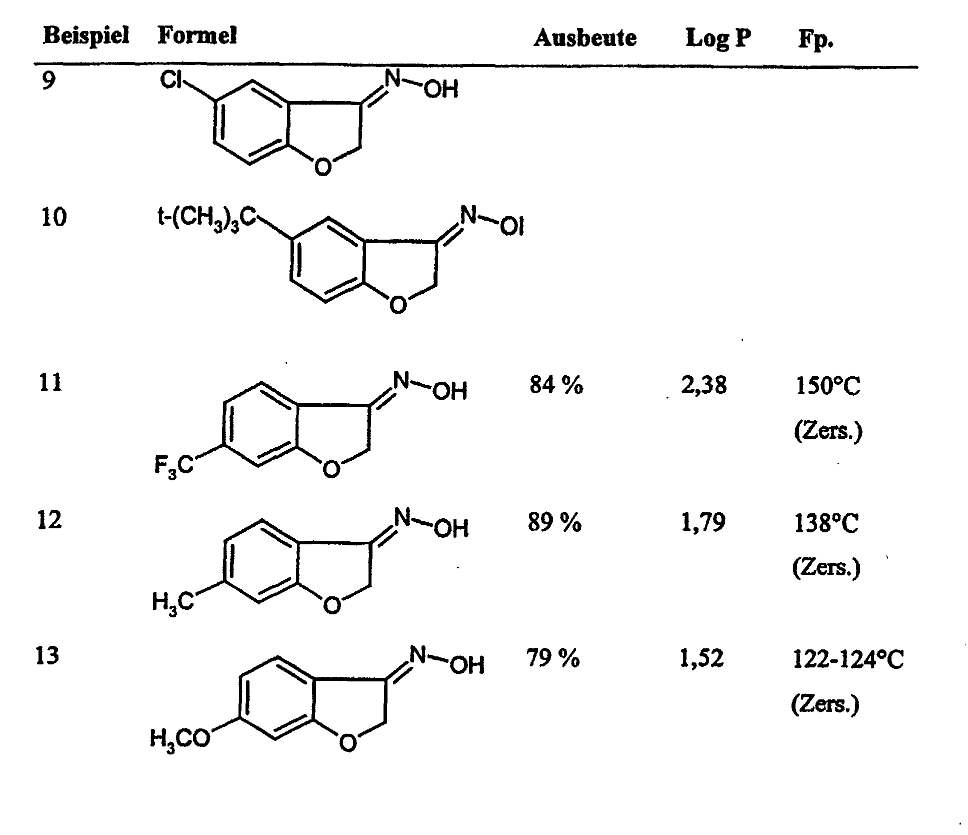

## Patentansprüche

1. Verfahren zur Herstellung von Verbindungen der Formel (I), 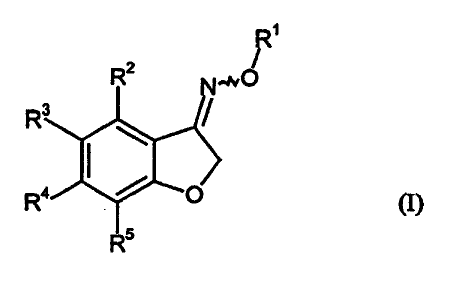 in welcher
R¹ für Wasserstoff oder Alkyl steht und
R², R³, R⁴ und R⁵ gleich oder verschieden sind und unabhängig voneinander für Wasserstoff, Alkyl, Alkoxy, Halogenalkyl oder Halogen steht,
**dadurch gekennzeichnet, dass** man Verbindungen der Formel (II), 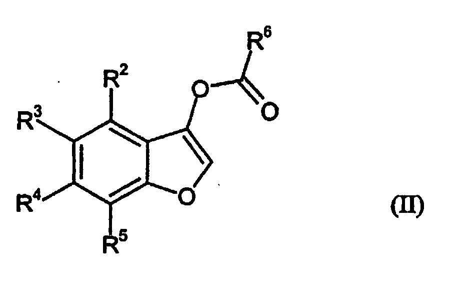 in welcher
R², R³, R⁴ und R⁵ die oben angegebenen Bedeutungen haben und
R⁶ für Alkyl steht,
mit einer Säure, gegebenenfalls in Gegenwart eines Verdünnungsmittels, hydrolysiert und das dabei erhaltene Benzfüranon der Formet (III), 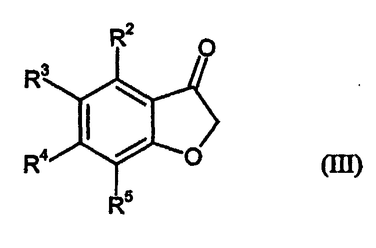 in welcher
R², R³, R⁴ und R⁵ die oben angegebenen Bedeutungen haben,
ohne Aufarbeitung mit einem Hydroxylaminderivat der Formel (IV),
H₂N-O-R¹ (IV)
in welcher
R¹ die oben angegebene Bedeutung hat,
oder einem Säureadditionskomplex davon,
gegebenenfalls in Gegenwart eines Verdünnungsmittels und gegebenenfalls in Gegenwart eines Puffermediums, umsetzt.

2. Verfahren gemäß Anspruch 1, **dadurch gekennzeichnet, dass** die zweite Stufe der Reaktion bei einem pH-Wert von 3 bis 7 in Essigsäure/Natriumacetat als Puffergemisch durchgeführt wird.

3. Verfahren gemäß den Ansprüchen 1 oder 2, **dadurch gekennzeichnet, dass** Hydroxylamin oder dessen Säureadditionskomplex verwendet wird.

4. Verfahren gemäß den Ansprüchen 1, 2 oder 3, **dadurch gekennzeichnet, dass** Methanol als Lösungsmittel verwendet wird.

5. Verfahren zur Herstellung von Verbindungen der Formel (II), 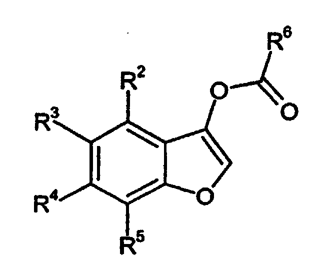 in welcher
R², R³, R⁴ und R⁵ gleich oder verschieden sind und unabhängig voneinander für Wasserstoff, Alkyl, Alkoxy, Halogenalkoxy oder Halogen steht, und
R⁶ für Alkyl steht,
**dadurch gekennzeichnet, dass** man Verbindungen der Formel (V), 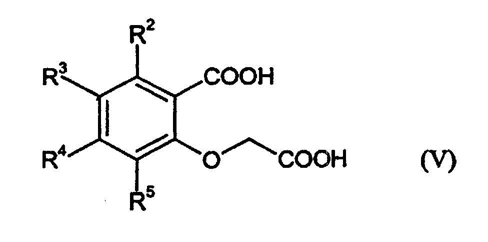 in welcher
R², R³, R⁴, R⁵ die oben angegebenen Bedeutungen haben,
mit aliphatischen Säureanhydriden der Formel (VI) 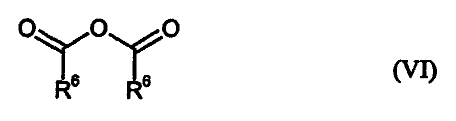 in welcher
R⁶ die oben angegebene Bedeutung hat,
oder aliphatischen Säurechloriden der Formel (VII), 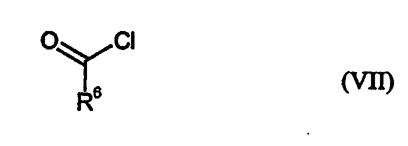 in welcher
R⁶ die oben angegebene Bedeutung hat,
in Gegenwart eines Katalysators umsetzt.

6. Verfahren gemäß Anspruch 5, **dadurch gekennzeichnet, dass** als Katalysator ein Pyridinderivat verwendet wird.

7. Verfahren gemäß den Ansprüchen 5 oder 6, **dadurch gekennzeichnet, dass** die Umsetzung mit Acetanhydrid als Verbindung der Formel (V) durchgeführt wird.

8. Verfahren gemäß den Ansprüchen 5, 6 oder 7, **dadurch gekennzeichnet, dass** das Verfahren in einem Temperaturbereich von 120°C bis 150°C durchgeführt wird.

## Claims

1. Process for preparing compounds of the formula (I), 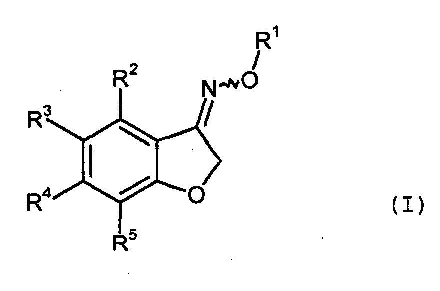 in which
R¹ represents hydrogen or alkyl and
R², R³, R⁴ and R⁵ are identical or different and independently of one another each represents hydrogen, alkyl, alkoxy, halogenoalkyl or halogen,
**characterized in that** compounds of the formula (II), 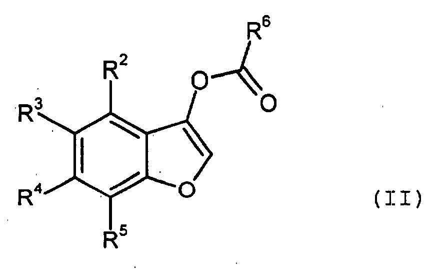 in which
R², R³, R⁴ and R⁵ are each as defined above
R⁶ represents alkyl
are hydrolyzed using an acid, if appropriate in the presence of a diluent, and the resulting benzofuranone of the formula (III), 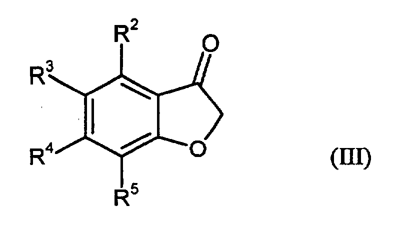 in which
R², R³, R⁴ and R⁵ are each as defined above
is reacted without work-up with a hydroxylamine derivative of the formula (IV),
H₂N-O-R¹ (IV)
in which
R¹ is as defined above,
or an acid addition complex thereof,
if appropriate in the presence of a diluent and if appropriate in the presence of a buffer medium.

2. Process according to Claim 1, **characterized in that** the second step of the reaction is carried out at a pH from 3 to 7 in acetic acid/sodium acetate as buffer mixture.

3. Process according to Claim 1 or 2, **characterized in that** hydroxylamine or its acid addition complex is employed.

4. Process according to any of Claims 1, 2 and 3, **characterized in that** the solvent used is methanol.

5. Process for preparing compounds of the formula (II), 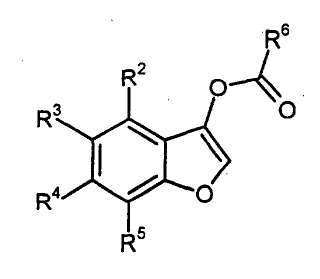 in which
R², R³, R⁴ and R⁵ are identical or different and independently of one another each represents hydrogen, alkyl, alkoxy, halogenoalkoxy or halogen, and
R⁶ represents alkyl,
**characterized in that** compounds of the formula (V), 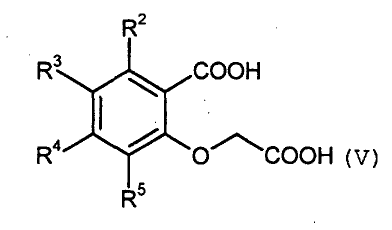 in which
R², R³, R⁴, R⁵ are each as defined above,
are reacted with aliphatic acid anhydrides of the formula (VI) 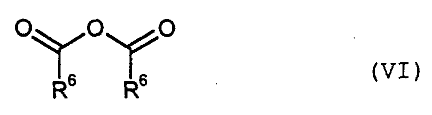 in which
R⁶ is as defined above
or aliphatic acyl chlorides of the formula (VII), 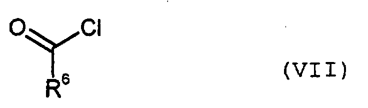 in which
R⁶ is as defined above
in the presence of a catalyst.

6. Process according to Claim 5, **characterized in that** the catalyst used is a pyridine derivative.

7. Process according to Claim 5 or 6, **characterized in that** the reaction is carried out using acetic anhydride as compound of the formula (V).

8. Process according to any of Claims 5, 6 and 7, **characterized in that** the process is carried out in a temperature range from 120°C to 150°C.

## Revendications

1. Procédé de production de composés de formule (I) 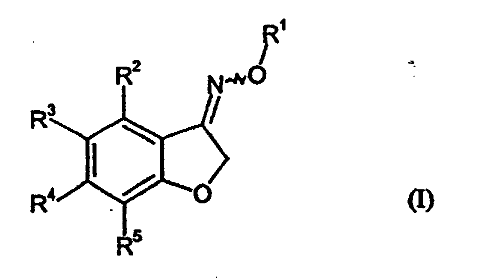 dans laquelle
R¹ représente l'hydrogène ou un reste alkyle et
R², R³, R⁴ et R⁴ sont identiques ou différents et représentent, indépendamment l'un de l'autre, l'hydrogène, un reste alkyle, alkoxy, halogénalkyle ou un halogène,
**caractérisé en ce qu'**on hydrolyse avec un acide, éventuellement en présence d'un diluant, des composés de formule (II) 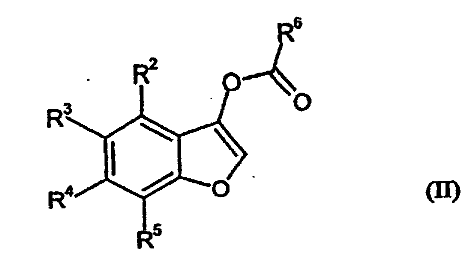 dans laquelle
R², R³, R⁴ et R⁵ ont les définitions indiquées ci-dessus et
R⁶ est un reste alkyle,
et on fait réagir la benzofurannone ainsi obtenue de formule (III) 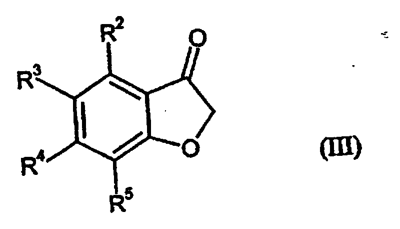 dans laquelle
R², R³, R⁴ et R⁵ ont les définitions indiquées ci-dessus,
sans autre traitement, avec un dérivé d'hydroxylamine de formule (IV)
H₂N-O-R¹ (IV)
dans laquelle
R¹ a la définition indiquée ci-dessus,
ou un complexe d'addition d'acide de ce dérivé,
le cas échéant en présence d'un diluant et en présence éventuelle d'un milieu tampon.

2. Procédé suivant la revendication 1, **caractérisé en ce que** la seconde étape de la réaction est conduite à un pH de 3 à 7 dans un mélange tampon formé d'acide acétique et d'acétate de sodium.

3. Procédé suivant la revendication 1 ou 2, **caractérisé en ce qu'**on utilise l'hydroxylamine ou un complexe d'addition d'acide de l'hydroxylamine.

4. Procédé suivant la revendication 1, 2 ou 3, **caractérisé en ce qu'**on utilise le méthanol comme solvant.

5. Procédé de production de composés de formule (II) 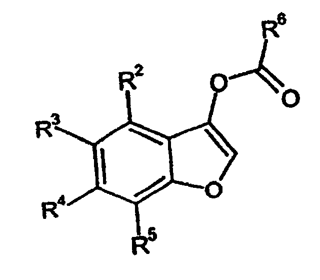 dans laquelle
R², R³, R⁴ et R⁵ sont identiques ou différents et représentent, indépendamment l'un de l'autre, l'hydrogène, un reste alkyle, alkoxy, halogénalkoxy ou un halogène, et
R⁶ est un reste alkyle,
**caractérisé en ce qu'**on fait réagir des composés de formule (V) 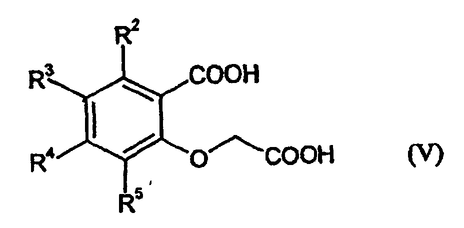 dans laquelle
R², R³, R⁴ et R⁵ ont les définitions indiquées ci-dessus,
avec des anhydrides d'acides aliphatiques de formule (VI) 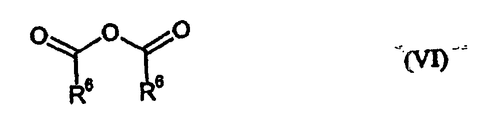 dans laquelle
R⁶ a la définition indiquée ci-dessus,
ou des chlorures d'acides aliphatiques de formule (VII) 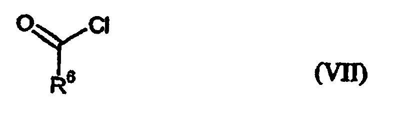 dans laquelle
R⁶ a la définition indiquée ci-dessus, en présence d'un catalyseur.

6. Procédé suivant la revendication 5, **caractérisé en ce qu'**on utilise comme catalyseur un dérivé de pyridine.

7. Procédé suivant la revendication 5 ou 6, **caractérisé en ce qu'**on conduit la réaction en utilisant l'acétanhydride comme composé de formule (V).

8. Procédé suivant les revendications 5, 6 ou 7, **caractérisé en ce qu'**il est mis en oeuvre dans une plage de températures de 120°C à 150°C.
